(19) Europäisches Patentamt
European Patent Office
Office européen des brevets

(11) Veröffentlichungsnummer : **0 463 996 A2**

# (12) EUROPÄISCHE PATENTANMELDUNG

(21) Anmeldenummer : **91810481.1**

(22) Anmeldetag : **20.06.91**

(51) Int. Cl.[5] : **C09B 29/44**

(30) Priorität : **29.06.90 CH 2172/90**

(43) Veröffentlichungstag der Anmeldung : **02.01.92 Patentblatt 92/01**

(84) Benannte Vertragsstaaten : **BE CH DE ES FR GB IT LI**

(71) Anmelder : **CIBA-GEIGY AG**
**Klybeckstrasse 141**
**CH-4002 Basel (CH)**

(72) Erfinder : **Walter, Harald, Dr.**
**Rufacherstrasse 20**
**CH-4055 Basel (CH)**
Erfinder : **Rigassi, Thomas**
**Weidstrasse 17C**
**CH-4800 Zofingen (CH)**

## (54) Azofarbstoffe, Verfahren zu deren Herstellung und deren Verwendung.

(57) Azofarbstoffe der Formel

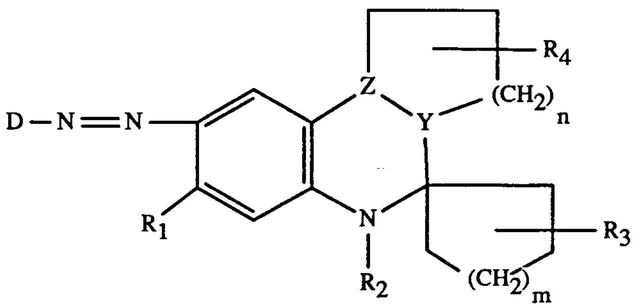

(1),

worin D eine Diazokomponente der Benzol- oder Naphthalinreihe oder der heterocyclischen Reihe ist, $R_1$ Wasserstoff, Halogen oder gegebenenfalls substituiertes $C_1$-$C_8$-Alkyl, $C_1$-$C_8$-Alkoxy, Phenoxy oder $C_2$-$C_8$-Alkanoylamino, $R_2$ Wasserstoff oder gegebenenfalls substituiertes $C_1$-$C_8$-Alkyl ist, $R_3$ und $R_4$ unabhängig voneinander Wasserstoff, Halogen oder gegebenenfalls substituiertes $C_1$-$C_8$-Alkyl oder Phenyl sind,

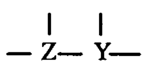

den Rest der Formel

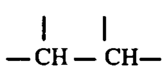

oder

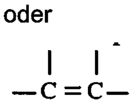

bedeutet und m und n unabhängig voneinander die Zahl 1 oder 2 sind, ergeben auf stickstoffhaltigen oder hydroxylgruppenhaltigen Fasermaterialien Färbungen von guten Echtheiten.

EP 0 463 996 A2

Die vorliegende Erfindung betrifft neue Azofarbstoffe, Verfahren zu deren Herstellung und Verwendung dieser Farbstoffe zum Färben und Bedrucken von Fasermaterialien, insbesondere textilen Fasermaterialien.

Gegenstand der vorliegenden Erfindung sind Azofarbstoffe der Formel

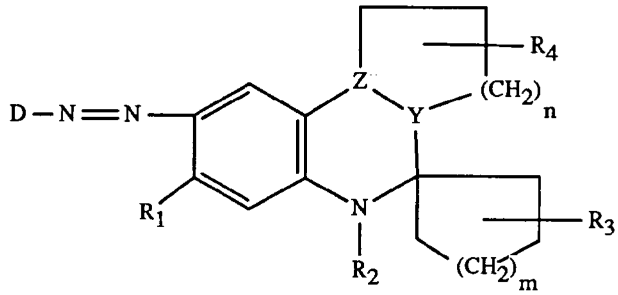

(1),

worin D eine Diazokomponente der Benzol- oder Naphthalinreihe oder der heterocyclischen Reihe ist, $R_1$ Wasserstoff, Halogen oder gegebenenfalls substituiertes $C_1$-$C_8$-Alkyl, $C_1$-$C_8$-Alkoxy, Phenoxy oder $C_2$-$C_8$-Alkanoylamino, $R_2$ Wasserstoff oder gegebenenfalls substituiertes $C_1$-$C_8$-Alkyl ist, $R_3$ und $R_4$ unabhängig voneinander Wasserstoff, Halogen oder gegebenenfalls substituiertes $C_1$-$C_8$-Alkyl oder Phenyl sind,

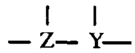

den Rest der Formel

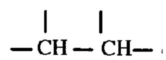

oder

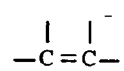

bedeutet und m und n unabhängig voneinander die Zahl 1 oder 2 sind.

Der rest D in Formel (1) kann die bei Diazokomponenten üblichen Substituenten enthalten, z.B. Alkylgruppen mit 1 bis 8, vorzugsweise 1 bis 4 Kohlenstoffatomen, wie z.B. Methyl, Aethyl, Propyl, Isopropyl, Butyl, Isobutyl, sek.-Butyl, tert.-Butyl, Hexyl, Octyl, wobei die Alkylgruppen durch Sulfo oder Sulfato substituiert sein können, Alkoxygruppen mit 1 bis 8 Kolhenstoffatomen, insbesondere 1 bis 4 Kohlenstoffatomen, wie z.B. Methoxy, Aethoxy, Propoxy, Isopropoxy, Butoxy, Acylaminogruppen wie Alkanoylaminogruppen mit 2 bis 8 Kohlenstoffatomen und Alkoxycarbonylaminogruppen mit 2 bis 8 Kohlenstoffatomen, wie z.B. Acetylamino, Propionylamino, Methoxycarbonyl-amino, Aethoxycarbonylamino, Alkanoylgruppen mit 2 bis 8, vorzugsweise 2 bis 4 Kohlenstoffatomen, wie z.B. Acetyl, Propionyl, Butyryl oder Isobutyryl, $C_5$-$C_7$-Cycloalkylcarbonyl, wie z.B. Cyclohexylcarbonyl, im Cycloalkylring durch $C_1$-$C_4$-Alkyl, wie z.B. Methyl, Aethyl, Propyl, Butyl, oder Halogen, wie z.B. Fluor, Chlor, Brom, Sulfo oder Sulfato, substituiertes $C_5$-$C_7$-Cycloalkylcarbonyl, Benzoyl, im Phenylring durch $C_1$-$C_4$-Alkyl, wie z.B. Methyl, Aethyl, Propyl, Butyl oder Halogen, wie z.B. Fluor, Chlor, Brom, Sulfo oder Sulfato, substituiertes Benzoyl, gegebenenfalls durch $C_1$-$C_4$-Alkyl, Halogen, Sulfo oder Sulfato substituiertes Benzthiazol oder Benzoxazol, Benzoylamino, Amino, Mono- oder Dialkylamino mit 1 bis 8 Kohlenstoffatomen im Alkylrest, Phenylamino, Alkoxycarbonyl mit 1 bis 8 Kohlenstoffatomen im Alkoxyrest, $C_5$-$C_7$-Cycloalkylaminosulfonyl, Nitro, Cyan, Trifluormethyl, Halogen, wie Fluor, Brom oder insbesondere Chlor, Sulfamoyl, am Stickstoffatom ein- oder zweifach durch $C_1$-$C_4$-Alkyl, $C_5$-$C_7$-Cycloalkyl, Phenyl oder Naphthyl substituiertes Sulfamoyl wobei die genannten Cyclohexyl-, Phenyl- und Naphthylringe des Sulfamoylrestes durch Sulfo, $C_1$-$C_4$-Alkyl oder Halogen substituiert sein können, Carbamoyl, Ureido, Hydroxy, $C_1$-$C_8$-Alkylsulfonyl, $C_1$-$C_8$-Alkylaminosulfonyl, gegebenenfalls im Phenylring durch $C_1$-$C_4$-Alkyl, Halogen wie z.B. Fluor, Chlor, Brom, Sulfo oder Sulfato substituiertes Phenylsulfonyl, Carboxy, Sulfomethyl, Sulfo, Sulfato, Thiosulfato, Arylazogruppen wie z.B. die Phenylazo- und Naphthylazogruppe sowie Phenyl, Naphthyl, Phenoxy, Phenoxysulfonyl, Phenylaminosulfonyl, wobei die angegebenen Phenyl- oder Naphthylreste durch die oben angegebenen Substituenten weitersubstituiert sein können. Gegebenenfalls können je zwei benachbarte Substituenten der genannten Ringsysteme weitere ankondensierte Phenylringe oder Cyclohexylringe bilden.

Als $C_1$-$C_8$-Alkyl kommt für $R_1$, $R_2$, $R_3$ und $R_4$ in Formel (1) z.B. in Betracht:

Methyl, Aethyl, Propyl, Isopropyl, Butyl, Isobutyl, sek.-Butyl, tert.-Butyl, Pentyl, Hexyl, Heptyl oder Octyl, sowie die entsprechenden Reste, die z.B. substituiert sind durch Hydroxy, Alkoxy mit 1 bis 4 Kohlenstoffatomen, wie z.B. Methoxy, Aethoxy, Propoxy, Isopropoxy oder Butoxy, Sulfo, Sulfato, Thiosulfato, Cyan oder Halogen, wie z.B. Fluor, Chlor oder Brom, oder Phenyl, wobei der Phenylrest durch die oben für D angegebenen Substituenten weitersubstituiert sein kann. Als Beispiele seien Trifluormethyl, Trichlormethyl, β-Hydroxyäthyl, β-Chloräthyl, β-Sulfatoäthyl und Benzyl genannt.

Als $C_1$-$C_8$-Alkoxy kommt für $R_1$ in Formel (1) z.B. in Betracht: Methoxy, Aethoxy, Propoxy, Isopropoxy, Butoxy, Isobutoxy, sek.-Butoxy, tert.-Butoxy, Hexyloxy und Octyloxy, sowie die entsprechenden Reste, die z.B. durch Alkoxy mit 1 bis 4 Kohlenstoffatomen substituiert sind, z.B. β-Methoxyäthoxy, β-Aethoxyäthoxy.

Als Phenoxy kommt für $R_1$ in Formel (1) ein unsubstituierter oder ein durch $C_1$-$C_4$-Alkyl, wie Methyl, Aethyl, Propyl, Isopropyl, Butyl, Isobutyl, sek.-Butyl und tert.-Butyl, $C_1$-$C_4$-Alkoxy, wie Methoxy, Aethoxy, Propoxy, Isopropoxy, Butoxy, Isobutoxy, sek.-Butoxy und tert.-Butoxy, Halogen, wie Fluor, Chlor und Brom, $C_2$-$C_4$-Alkanoylamino, wie Acetylamino und Propionylamino, Benzoylamino und Sulfo substituierter Phenoxy-rest in Betracht.

Als Phenyl kommt für $R_3$ und $R_4$ in Formel (1) unabhängig voneinander ein unsubstituierter oder ein durch $C_1$-$C_4$-Alkyl, wie Methyl, Aethyl, Propyl, Isopropyl, Butyl, Isobutyl, sek.-Butyl und tert.-Butyl, $C_1$-$C_4$-Alkoxy, wie Methoxy, Aethoxy, Propoxy, Isopropoxy, Butoxy, Isobutoxy, sek.-Butoxy und tert.-Butoxy, Halogen, wie Fluor, Chlor und Brom, $C_2$-$C_4$-Alkanoylamino, wie Acetylamino und Propionylamino, Benzoylamino und Sulfo substituierter Phenylrest in Betracht.

Als $C_2$-$C_8$-Alkanoylamino kommt für $R_1$ in Formel (1) z.B. in Betracht: Acetylamino, Propionylamino, sowie die entsprechenden Reste, die z.B. durch Sulfo oder Sulfato substituiert sind.

Als Halogen kommt für $R_1$, $R_3$ und $R_4$ in Formel (1) z.B. Fluor, Chlor oder Brom in Betracht.

Vorzugsweise haben in den Azofarbstoffen der Formel (1) $R_3$ und $R_4$ sowie m und n die gleichen Bedeutungen.

Bevorzugt sind Azofarbstoffe der Formel (1), worin D Thienyl, Thiazolyl, Isothiazolyl, 1,2,4-Thiadiazolyl, 1,3,4-Thiadiazolyl, Benzthiazolyl, Benzisothiazolyl, Benzoxazolyl, Pyrazolyl, Imidazolyl, 1,2,3-Triazolyl, 1,2,4-Triazolyl, Thiophenyl, Benzthiophenyl, Tetrahydrobenzthiophenyl, 7-Oxotetrahydrobenzo[b]thiophenyl, Pyridinyl, Indazolyl, Phenyl oder Naphthyl, insbesondere Phenyl, ist, wobei die angegebenen Reste durch die oben für D angegebenen Substituenten weitersubstituiert sein können.

Bevorzugt sind ferner Azofarbstoffe der Formel (1), worin $R_1$ Wasserstoff, $C_1$-$C_4$-Alkyl, insbesondere Methyl, oder $C_1$-$C_4$-Alkoxy, insbesondere Methoxy, ist.

Bevorzugt sind Azofarbstoffe der Formel (1), worin $R_2$ Wasserstoff oder gegebenenfalls durch Phenyl substituiertes $C_1$-$C_4$-Alkyl, insbesondere Aethyl, ist. In der Bedeutung als durch Phenyl substituiertes $C_1$-$C_4$-Alkyl ist $R_2$ vorzugsweise Benzyl.

Bevorzugt sind weiterhin Azofarbstoffe der Formel (1), worin $R_3$ und $R_4$ unabhängig voneinander Wasserstoff oder gegebenenfalls substituiertes Phenyl sind.

Bevorzugt sind ebenfalls Azofarbstoffe der Formel (1), worin n und m beide die Zahl 1 oder n und m beide die Zahl 2 bedeuten.

Von besonderem Interesse sind Azofarbstoffe der Formel (1), worin

$$-\overset{|}{Z}-\overset{|}{Y}-$$

den Rest der Formel

$$-\overset{|}{CH}-\overset{|}{CH}-$$

bedeutet.

Besonders bevorzugt sind Azofarbstoffe der Formel (1), worin $R_1$ Wasserstoff, $C_1$-$C_4$-Alkyl oder $C_1$-$C_4$-Alkoxy, $R_2$ Wasserstoff oder gegebenenfalls durch Phenyl substituiertes $C_1$-$C_4$-Alkyl ist und $R_3$ und $R_4$ unabhängig voneinander Wasserstoff oder gegebenenfalls substituiertes Phenyl sind.

Ganz besonders bevorzugt sind Azofarbstoffe der Formel (1), worin $R_1$ Wasserstoff, Methyl oder Methoxy, $R_2$ Wasserstoff oder Äthyl und $R_3$ und $R_4$ unabhängig voneinander Wasserstoff oder Phenyl sind. Vorzugsweise bedeutet in den ganz besonders bevorzugten Azofarbstoffen der Formel (1)

$$-\overset{|}{Z}-\overset{|}{Y}-$$

den Rest der Formel

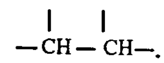

Gegenstand der Erfindung ist ferner ein Verfahren zur Herstellung der Azofarbstoffe der Formel (1), welches dadurch gekennzeichnet ist, dass man ein Amin der Formel

$$D\text{-}NH_2 \quad (2)$$

diazotiert und auf eine Kupplungskomponente der Formel

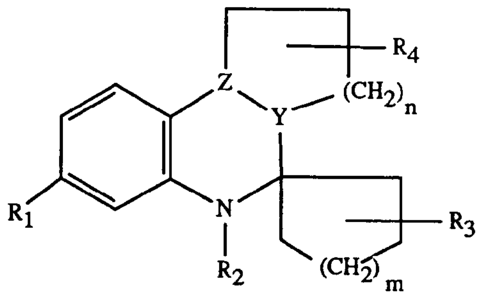

(3)

kuppelt, wobei D, $R_1$, $R_2$, $R_3$, $R_4$,

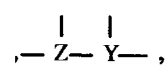

n und m die unter Formel (1) angegebenen Bedeutungen haben.

Gegebenenfalls kann nach der Kupplung noch eine wasserlöslichmachende Gruppe in den Azofarbstoff der Formel (1) eingeführt werden; ferner kann im Anschluss an die Kupplung noch eine Alkylierung, Acylierung sowie eine Sulfatoesterbildung vorgenommen werden.

Die Diazotierung der Diazokomponente der Formel (2) erfolgt in der Regel durch Einwirken salpetriger Säure in wässrig-mineralsaurer Lösung bei tiefer Temperatur, die Kupplung auf die Kupplungskomponente der Formel (3) bei sauren, neutralen bis alkalischen pH-Werten.

Gegebenenfalls kann eine freie Aminogruppe im Rest D nach der Kupplung mit einem Acylierungs- oder Alkylierungsmittel in eine Acylamino- oder Alkylaminogruppe umgewandelt werden, und ebenso kann eine geeignete Hydroxygruppe durch Acylierung oder Alkylierung in eine Acyloxy- oder Alkoxygruppe überführt werden.

Ferner kann eine freie aliphatische Hydroxygruppe in eine wasserlöslichmachende Gruppe, wie z.B. durch Sulfatierung in eine Sulfatogruppe, überführt werden.

Vorzugsweise werden für das erfindungsgemässe Verfahren Amine der Formel (2) verwendet, worin D Thienyl, Thiazolyl, Isothiazolyl, 1,2,4-Thiadiazolyl, 1,3,4-Thiadiazolyl, Benzthiazolyl, Benzisothiazolyl, Benzoxazolyl, Pyrazolyl, Imidazolyl, 1,2,3-Triazolyl, 1,2,4-Triazolyl, Thiophenyl, Benzthiophenyl, Tetrahydrobenzthiophenyl, 7-Oxotetrahydrobenzo[b]thiophenyl, Pyridinyl, Indazolyl, Phenyl oder Naphthyl, insbesondere Phenyl, ist, wobei die angegebenen Reste durch die oben für D angegebenen Substituenten weitersubstituiert sein können.

Bevorzugt für das erfindungsgemässe Verfahren sind Kupplungskomponenten der Formel (3), worin $R_1$ Wasserstoff, $C_1$-$C_4$-Alkyl, insbesondere Methyl, oder $C_1$-$C_4$-Alkoxy, insbesondere Methoxy, ist.

Bevorzugt für das erfindungsgemässe Verfahren sind ferner Kupplungskomponenten der Formel (3), worin $R_2$ Wasserstoff oder gegebenenfalls durch Phenyl substituiertes $C_1$-$C_4$-Alkyl, insbesondere Aethyl, ist. In der Bedeutung als durch Phenyl substituiertes $C_1$-$C_4$-Alkyl ist $R_2$ vorzugsweise Benzyl.

Bevorzugt für das erfindungsgemässe Verfahren sind weiterhin Kupplungskomponenten der Formel (3), worin $R_3$ und $R_4$ unabhängig voneinander Wasserstoff oder gegebenenfalls substituiertes Phenyl sind.

Vorzugsweise sind in den Kupplungskomponenten der Formel (3) n und m beide die Zahl 1 oder n und m beide die Zahl 2, insbesondere haben $R_3$ und $R_4$ sowie m und n die gleichen Bedeutungen.

Von besonderem Interesse sind Kupplungskomponenten der Formel (3), worin

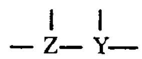

den Rest der Formel

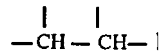

bedeutet.

Besonders bevorzugt für das erfindungsgemässe Verfahren sind Kupplungskomponenten der Formel (3), worin $R_1$ Wasserstoff, $C_1$-$C_4$-Alkyl oder $C_1$-$C_4$-Alkoxy, $R_2$ Wasserstoff oder gegebenenfalls durch Phenyl subsrituiertes $C_1$-$C_4$-Alkyl ist und $R_3$ und $R_4$ unabhängig voneinander Wasserstoff oder gegebenenfalls substituiertes Phenyl sind.

Ganz besonders bevorzugt für das erfindungsgemässe Verfahren sind Kupplungskomponenten der Formel (3), worin $R_1$ Wasserstoff, Methyl oder Methoxy, $R_2$ Wasserstoff oder Äthyl und $R_3$ und $R_4$ unabhängig voneinander Wasserstoff oder Phenyl sind, wobei

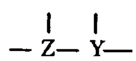

vorzugsweise den Rest der Formel

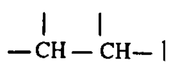

bedeutet.

Die Amine der Formel (2) sind an sich bekannt und können in Analogie zu bekannten Verbindungen hergestellt werden.

Die Amine der Formel (2) können in Aminogruppen überführbare Reste, wie z.B. die Acetylamino- und die Nitrogruppe, enthalten. Beispielsweise kann eine Acetylaminogruppe durch Verseifen und eine Nitrogruppe durch Reduktion in eine Aminogruppe überführt werden, vorteilhafterweise im Anschluss an die Herstellung der Farbstoffe der Formel (1).

Ferner kann eine wasserlöslichmachende Gruppe im Anschluss an Diazotierung und Kupplung in die Azofarbstoffe eingeführt werden, z.B. durch Sulfatierung einer Hydroxygruppe im Amin der Formel (2).

Die Verbindungen der Formel (3) sind zum Teil neu. Die neuen Verbindungen der Formel (3) stellen einen weiteren Gegenstand der Erfindung dar.

Gegenstand der vorliegenden Erfindung sind somit ferner Verbindungen der Formel

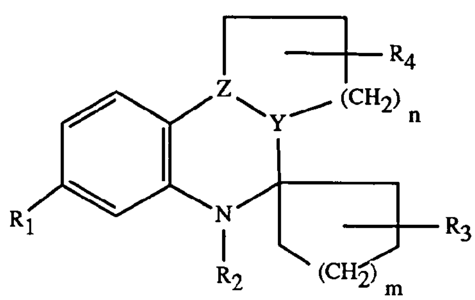

(4),

worin

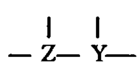

den Rest der Formel

$$-\overset{|}{C}H-\overset{|}{C}H-$$

bedeutet, $R_1$ Wasserstoff, Halogen oder gegebenenfalls substituiertes $C_1$-$C_8$-Alkyl, $C_1$-$C_8$-Alkoxy, Phenoxy oder $C_2$-$C_8$-Alkanoylamino, $R_2$ Wasserstoff oder gegebenenfalls substituiertes $C_1$-$C_8$-Alkyl ist, $R_3$ und $R_4$ unabhängig voneinander Wasserstoff, Halogen oder gegebenenfalls substituiertes $C_1$-$C_8$-Alkyl oder Phenyl und m und n unabhängig voneinander die Zahl 1 oder 2 sind, oder worin

$$-\overset{|}{Z}-\overset{|}{Y}-$$

den Rest der Formel

$$-\overset{|}{C}=\overset{|}{C}-$$

bedeutet, $R_1$ Halogen oder gegebenenfalls substituiertes $C_1$-$C_8$-Alkyl, $C_1$-$C_8$-Alkoxy, Phenoxy oder $C_2$-$C_8$-Alkanoylamino ist und $R_2$, $R_3$, $R_4$, m und n die unter Formel (4) angegebenen Bedeutungen haben.

Bevorzugt sind Verbindungen der Formel (4), worin

$$-\overset{|}{Z}-\overset{|}{Y}-$$

den Rest der Formel

$$-\overset{|}{C}H-\overset{|}{C}H-$$

bedeutet und $R_1$ Wasserstoff, $C_1$-$C_4$-Alkyl, insbesondere Methyl, oder $C_1$-$C_4$-Alkoxy, insbesondere Methoxy, ist, oder worin

$$-\overset{|}{Z}-\overset{|}{Y}-$$

den Rest der Formel

$$-\overset{|}{C}=\overset{|}{C}-$$

bedeutet und $R_1$ $C_1$-$C_4$-Alkyl, insbesondere Methyl, oder $C_1$-$C_4$-Alkoxy, insbesondere Methoxy, ist.

Vorzugsweise ist in den Verbindungen der Formel (4) $R_2$ Wasserstoff oder gegebenenfalls durch Phenyl substituiertes $C_1$-$C_4$-Alkyl, insbesondere Aethyl. In der Bedeutung als durch Phenyl substituiertes $C_1$-$C_4$-Alkyl ist $R_2$ vorzugsweise Benzyl.

Bevorzugt sind weiterhin Verbindungen der Formel (4), worin $R_3$ und $R_4$ unabhängig voneinander Wasserstoff oder gegebenenfalls substituiertes Phenyl sind.

Ebenfalls bevorzugt sind Verbindungen der Formel (4), worin n und m beide die Zahl 1 oder n und m beide die Zahl 2 bedeuten.

Von besonderem Interesse sind Verbindungen der Formel (4), worin

$$-\overset{|}{Z}-\overset{|}{Y}-$$

den Rest der Formel

$$-\overset{|}{C}H-\overset{|}{C}H-$$

bedeutet.

Besonders bevorzugt sind Verbindungen der Formel (4), worin $R_2$ Wasserstoff oder gegebenenfalls durch Phenyl substituiertes $C_1$-$C_4$-Alkyl ist und $R_3$ und $R_4$ unabhängig voneinander Wasserstoff oder gegebenenfalls substituiertes Phenyl sind.

Ganz besonders bevorzugt sind Verbindungen der Formel (4), worin $R_1$ Wasserstoff, Methyl oder Methoxy, $R_2$ Wasserstoff oder Äthyl ist, $R_3$ und $R_4$ unabhängig voneinander Wasserstoff oder Phenyl sind und

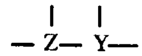

den Rest der Formel

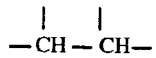

bedeutet.

Ein weiterer Gegenstand der Erfindung ist ein Verfahren zur Herstellung der Verbindungen der Formel (4), welches dadurch gekennzeichnet ist, dass man eine Verbindung der Formel

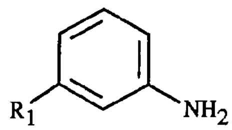

(5),

mit den Verbindungen der Formeln

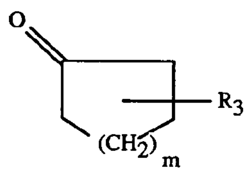

(6) und

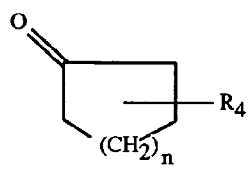

(7)

oder mit einer Verbindung der Formel

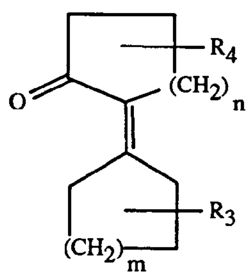

(8)

zu einer Verbindung der Formel

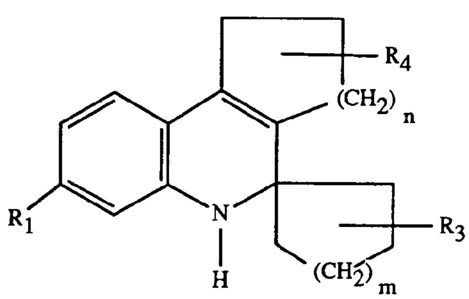

(9)

umsetzt, gegebenenfalls die Verbindung der Formel (9) zu einer Verbindung der Formel

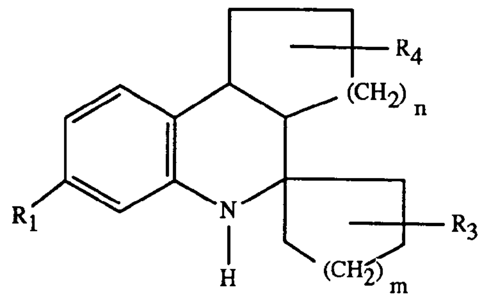

(10)

hydriert und gegebenenfalls die Verbindung der Formel (9) oder (10) mit einer Verbindung der Formel

$(R_2)_2X$ (11)

oder

$R_2X$ (12),

worin X eine ein- oder zweiwertige anionische Abgangsgruppe ist, umsetzt, wobei $R_1$, $R_2$, $R_3$, $R_4$, n und m die unter Formel (4) angegebenen Bedeutungen haben.

Als X in Formel (11) kommt z.B. Sulfat in Betracht. Als Beispiele für Verbindungen der Formel (11) seien Diäthylsulfat oder Dimethylsulfat genannt. Als X in Formel (12) kommt z.B. Halogen, wie Fluor, Chlor oder Brom, in Betracht.

Die Umsetzung der Verbindung der Formel (5) mit den Verbindungen der Formeln (6) und (7) sowie insbesondere die Umsetzung der Verbindung der Formel (5) mit der Verbindung der Formel (8) erfolgt in organischen Lösungsmitteln, wie z.B. Toluol, Xylol, Chlorbenzol oder Dichlorbenzol, in Gegenwart einer Säure, wie z.B. Salzsäure, Schwefelsäure oder p-Toluolsulfonsäure, bei Temperaturen von z.B. 90 bis 190°C, wobei im Verlaufe der Umsetzung gebildetes Wasser z.B. mit einem Wasserabscheider abgetrennt werden kann.

In einer weiteren, bevorzugten Ausführungsform erfolgt die Umsetzung der Verbindung der Formel (5) mit den Verbindungen der Formeln (6) und (7) oder mit der Verbindung der Formel (8) in Gegenwart von Jod, bei einer Temperatur von z.B. 90 bis 190°C, in einem Lösungsmittel, wie z.B. Toluol, Xylol, Chlorbenzol oder Dichlorbenzol, oder ohne Lösungsmittel. Während der Umsetzung gebildetes Wasser kann z.B. im Verlauf der Reaktion direkt abdestilliert werden. Für die Umsetzung in Gegenwart von Jod wird die Verbindung der Formel (5) vorzugsweise ohne Lösungsmittel vorgelegt.

Die Hydrierung der Verbindung der Formel (9) erfolgt in einem organischen Lösungsmittel, wie z.B. Methanol, Aethanol oder Tetrahydrofuran, in Gegenwart eines Katalysators, wie z.B. feinverteiltem Nickel, Platin-/Kohle oder Palladium/Kohle, ohne Druck oder unter einem Druck von z.B. 1 bis 50 bar, insbesondere 4 bis 6 bar, bei einer Temperatur von z.B. 20 bis 120°C.

Die Umsetzung der Verbindung der Formel (9) oder (10) mit einer Verbindung der Formel (11) oder (12) erfolgt in einem organischen Lösungmittel, wie z.B. Isopropanol, Toluol, Xylol oder Chlorbenzol, in Gegenwart eines Erdalkalioxids, wie z.B. Magnesiumoxid, oder eines Alkalicarbonats, wie z.B. Natrium- oder Kaliumcarbonat, bei Temperaturen von z.B. 70 bis 170°C.

Bevorzugte Ausführungsformen des erfindungsgemässen Verfahrens zur Herstellung der Verbindungen der Formel (4) sind dadurch gekennzeichnet, dass man

– eine Verbindung der Formel (5) verwendet, worin

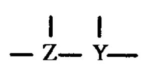

den Rest der Formel

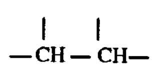

bedeutet und $R_1$ Wasserstoff, $C_1$-$C_4$-Alkyl, insbesondere Methyl, oder $C_1$-$C_4$-Alkoxy, insbesondere Methoxy, ist, oder worin

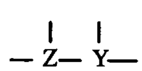

den Rest der Formel

$$-\overset{|}{C}=\overset{|}{C}-$$

bedeutet und $R_1$ $C_1$-$C_4$-Alkyl, insbesondere Methyl, oder $C_1$-$C_4$-Alkoxy, insbesondere Methoxy, ist.
– Verbindungen der Formeln (6) und (7) oder eine Verbindung der Formel (8) verwendet, worin $R_3$ und $R_4$ unabhängig voneinander Wasserstoff oder gegebenenfalls substituiertes Phenyl sind.
– die Verbindung der Formel (9) durch Hydrierung zur Verbindung der Formel (10) umsetzt;
– als Verbindung der Formel (11) Diäthylsulfat verwendet.

Die Farbstoffe der Formel (1) liegen entweder in der Form ihrer freien Säure oder vorzugsweise als deren Salze vor.

Als Salze kommen beispielsweise die Alkali- oder Ammmoniumsalze oder die Salze eines organischen Amins in Betracht.

Als Beispiel seien die Natrium-, Lithium-, Kalium- oder Ammoniumsalze oder das Salz des Mono-, Di- oder Triäthanolamins genannt.

Die erfindungsgemässen Azofarbstoffe der Formel (1) eignen sich nach an sich bekannten Methoden zum Färben und Bedrucken, insbesondere von stickstoffhaltigen oder hydroxygruppenhaltigen Fasermaterialien, wie z.B. textilen Fasermaterialien aus Cellulose, Polyester, Seide und insbesondere Wolle und synthetischen Polyamiden. Man erhält egale Färbungen mit guten Allgemeinechtheiten, insbesondere guter Reib-, Nass-, Nassreib- und Lichtechtheit. Ferner sind die erfindungsgemässen Farbstoffe gut mit anderen Farbstoffen kombinierbar. Das oben genannte Textilmaterial kann in den verschiedensten Verarbeitungsformen vorliegen, wie z.B. als Faser, Gam, Gewebe oder Gewirke.

In den folgenden Beispielen stehen Teile für Gewichtsteile. Die Temperaturen sind Celsiusgrade. Die Beziehung zwischen Gewichtsteilen und Volumenteilen ist dieselbe wie diejenige zwischen Gramm und Kubikzentimeter.

Beispiel 1: In einem Sulfierkolben werden 108,2 Teile 1,3-Toluidin und 2,5 Teile Jod unter Rühren vorgelegt. Danach wird die Innentemperatur auf 150 bis 155° erhöht und es werden 186,9 Teile Cyclopentanon innerhalb 2 Stunden so zugetropft, dass die Innentemperatur nicht unter 140° sinkt. Das während der Reaktion gebildete Reaktionswasser wird direkt über eine an die Apparatur angesetzte Destillationsbrücke abdestilliert. Nach dem Zutropfen des Cyclopentanons wird noch 2,5 Stunden bei 160° gerührt und anschliessend wird im Hochvakuum desrilliert. Die bei einer Temperatur von 175 bis 205° und einem Druck von $10^{-1}$ mbar siedende Fraktion stellt das Rohprodukt dar, das durch Umkristallisation aus wenig Petrolether (50/70) weiter gereinigt werden kann. Man erhält 135 Teile einer Festsubstanz, die der Verbindung der Formel

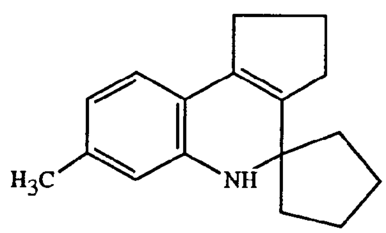

(101)

entspricht. Die Verbindung der Formel (101) weist eine gaschromatographisch ermittelte Reinheit von 96% auf.

Beispiele 2 und 3: Verfährt man wie in Beispiel 1 angegeben, verwendet jedoch statt 108,2 Teile 1,3-Toluidin eine äquimolare Menge Anilin oder 3-Methoxyanilin, so erhält man die Verbindungen der Formeln

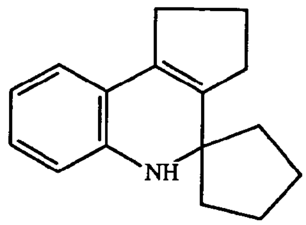

(102) und

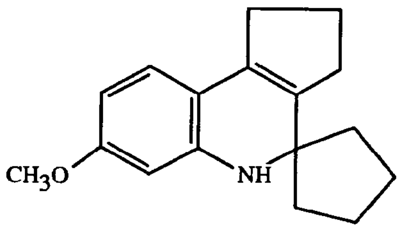

(103),

mit einem Schmelzpunkt der Verbindung der Formel (102) von 55 bis 56° und einem Schmelzpunkt der Verbindung der Formel (103) von 113 bis 116°.

Beispiel 4: In einem Sulfierkolben werden 62,8 Teile 3-Methoxyanilin, 116 Teile Cyclopentylidencyclopentanon und 2 Teile para-Toluolsulfonsäure in 200 Teile absolutem Toluol vorgelegt. Die Reaktionsmischung wird anschliessend 15 Stunden bei einer Innentemperatur von 120 bis 125° am Wasserabscheider gerührt und nach Abkühlen mit einer wässrigen Natriumhydroxid-Lösung (30%) neutralisiert. Die Reaktionslösung wird zweimal mit je 100 Teilen 5 %-iger Natriumchlorid-Lösung gewaschen und danach über Natriumsulfat getrocknet. Nach dem Abziehen des Toluols im Wasserstrahlvakuum wird im Hochvakuum destilliert. Man erhält 91,4 Teile eines Rohprodukts, das bei einer Temperatur von 145 bis 190° und einem Druck von 5- $10^{-2}$ mbar siedet. Nach längerem Stehenlassen wird das erhaltene Produkt fest. Zur weiteren Reinigung wird das erhaltene Produkt in Ether gelöst, durch Zufuhr von gasförmigem Chlorwasserstoff in das Hydrochlorid überführt und anschliessend aus kaltem Aceton umkristallisiert. Man erhält 64 Teile leicht beiger Kristalle, die der Verbindung der Formel

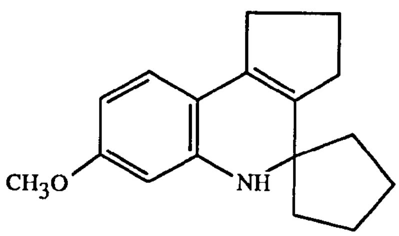

(103)

entsprechen. Die wie oben angegeben erhaltene Verbindung der Formel (103) weist eine gaschromatographisch ermittelte Reinheit von 91 % auf.

Beispiele 5 und 6: Verfährt man wie in Beispiel 4 angegeben, verwendet jedoch statt 62,8 Teile 3-Methoxyanilin eine äquimolare Menge 1,3-Toluidin oder Anilin, so erhält man die Verbindungen der Formeln (101) und (102).

Beispiel 7: In einem Sulfierkolben werden 32,5 Teile 1,3-Toluidin und 1 Teil Jod vorgelegt und die Innentemperatur auf ca 160° erhöht. Eine Lösung von 115,6 Teilen 4-Phenylcyclohexanon und 350 Teilen Toluol wird anschliessend innerhalb ca. 3 Stunden bei einer Innentemperatur von 140 bis 150° unter Niveau zugetropft. Entstehendes Reaktionswasser wird dabei direkt aus dem Reaktionskolben abdestilliert. Nach Beendigung des Zutropfens wird ca. 3 Stunden nachgerührt. Nach dem Abkühlen auf Raumtemperatur wird ein Gemisch von 150 Teilen Ether und 200 Teilen Petrolether zugetropft und die Lösung vor der Filtration auf eine Temperatur von ca. -20° abgekühlt. Anschliessend wird das erhaltene Rohprodukt durch Umkristallisation aus kaltem Petrolether (50/70) weiter gereinigt. Man erhält 62,7 Teile eines weissen Pulvers, das der Verbindung der Formel

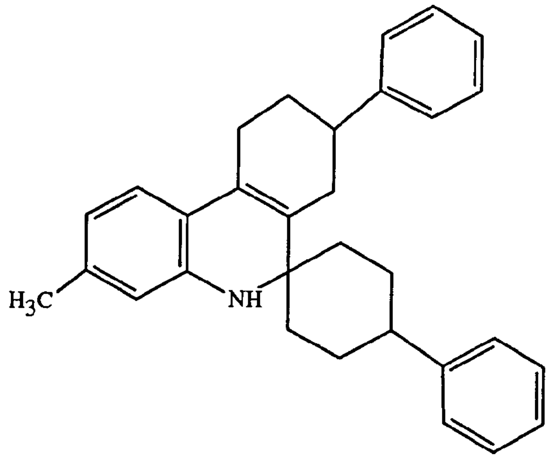

(104)

entspricht. Die erhaltene Verbindung der Formel ( 104) weist eine gaschromatographisch ermittelte Reinheit von 98% und einen Schmelzpunkt von 133 bis 134° auf.

Beispiele 8 bis 12: Verfährt man wie in Beispiel 7 angegeben, verwendet jedoch gegebenenfalls statt 32,5 Teile 1,3-Toluidin eine äquimolare Menge 3-Methoxyanilin oder Anilin und gegebenenfalls statt 115,6 Teile 4-Phenylcyclohexanon eine äquimolare Menge Cyclohexanon, so erhält man die in Tabelle 1 in Spalte 2 angegebenen Hexahydrophenanthridinverbindungen.

Tabelle 1

| Bsp. | Hexahydrophenanthridinverbindung | |
|---|---|---|
| 8 | 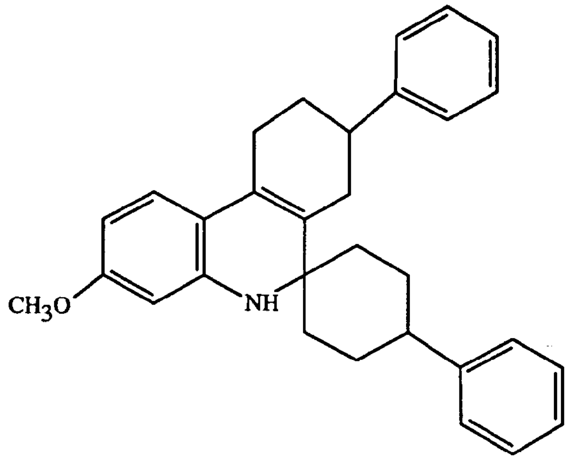 | (105) |
| 9 | 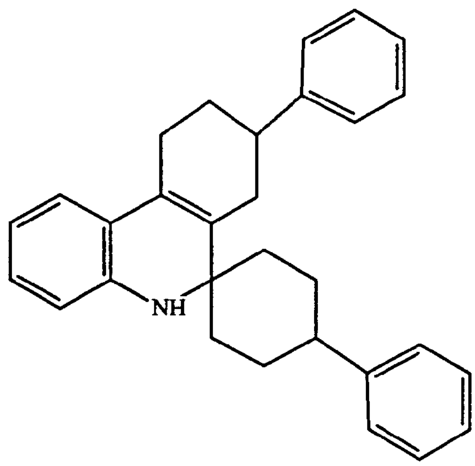 | (106) |
| 10 | 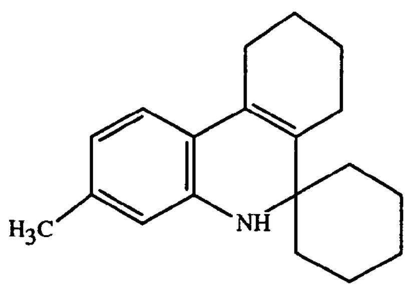 | (107) |

| Bsp. | Hexahydrophenanthridinverbindung | |
|---|---|---|
| 11 | 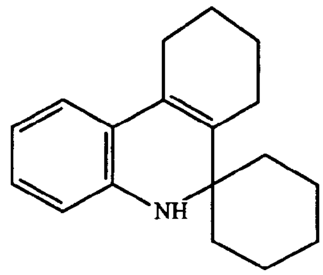 | (108) |
| 12 | 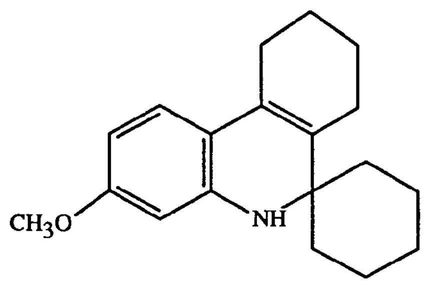 | (109) |

Beispiel 13: 184,6 Teile der gemäss Beispiel 10 hergestellten Verbindung der Formel (107), welche in einer Reinheit von 90% vorliegt, werden in 1800 Teilen absolutem Aethanol gelöst. Nach Zugabe von 74 Teilen Raney-Nickel wird 8,5 Stunden bei einer Temperatur von 50 bis 60° und einem Druck von 5 bar hydriert. Nach der theoretischen Aufnahme der Wasserstoffmenge wird die Reaktion abgebrochen und der Katalysator abfiltriert. Die Reaktionslösung wird anschliessend im Wasserstrahlvakuum konzentriert und das zurückbleibende Öl in 200 Teilen absolutem Aether gelöst. Nach Abkühlen auf eine Temperatur von 0° werden 26,6 Teile gasförmigen Chlorwasserstoffs innerhalb einer Stunde so eingeleitet, dass die Innentemperatur 5° nicht übersteigt. Danach wird kalt abgesaugt und mit wenig Aether nachgewaschen. Nach Trocknung an der Luft erhält man 181 Teile eines weissen Pulvers, das der Verbindung der Formel

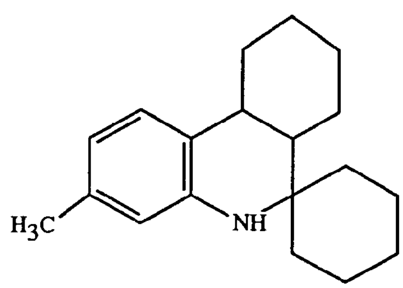

(110)

entspricht, die als Hydrochlorid vorliegt. Die als Hydrochlorid vorliegende Verbindung der Formel (110) weist eine flüssigchromatographisch bestimmte Reinheit von 96,5% auf.

Beispiele 14 bis 21: Verfährt man wie in Beispiel 13 angegeben, verwendet jedoch statt 184,6 Teile der gemäss Beispiel 10 hergestellten Verbindung der Formel (107) eine äquimolare Menge einer der gemäss den Beispielen 4 bis 9 oder 11 oder 12 erhaltenen Verbindungen, so erhält man die in Tabelle 2 in Spalte 2 angegebenen Octahydrophenanthridinverbindungen bzw. die angegebenen mit Octahydrophenanthridin verwandten Verbindungen. Für einige dieser Verbindungen sind in Tabelle 2 in Spalte 3 die Siede- bzw. Schmelzpunkte angegeben.

Tabelle 2

| Bsp. | Octahydrophenanthridinverbindungen und verwandte Verbindungen | | Siede- bzw. Schmelzpunkt |
|---|---|---|---|
| 14 | 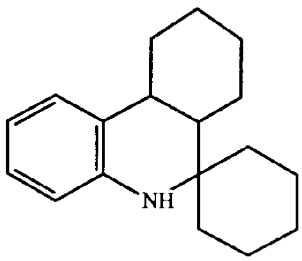 | (111) | |
| 15 | 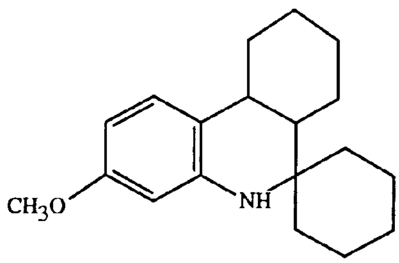 | (112) | Siedepunkt: 150-160° bei einem Druck von $10^{-1}$ mbar |
| 16 | 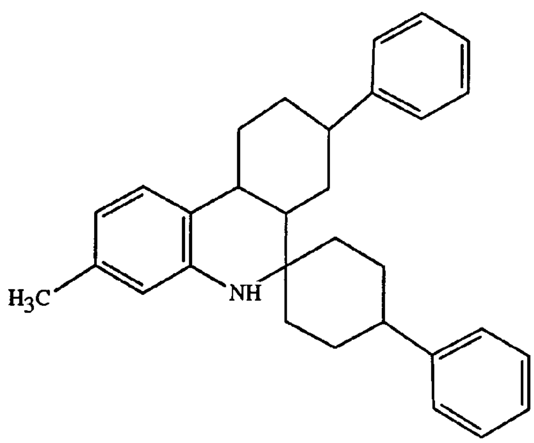 | (113) | Schmelzpunkt: 202-206° |

| Bsp. | Octahydrophenanthridinverbindungen und verwandte Verbindungen | | Siede- bzw. Schmelzpunkt |
|---|---|---|---|
| 17 | 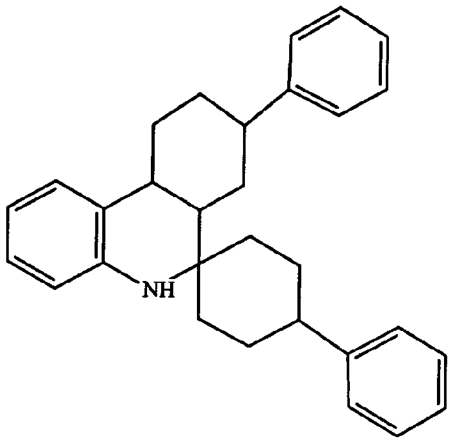 | (114) | |
| 18 | 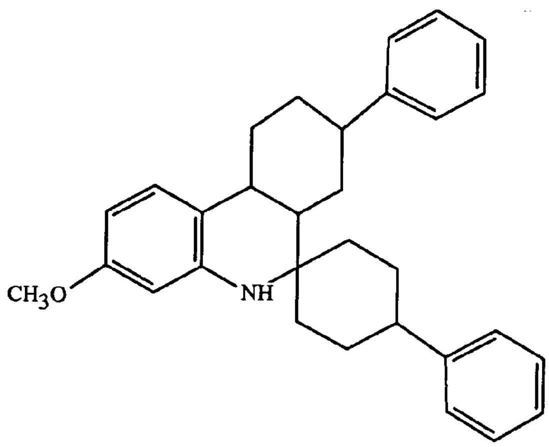 | (115) | |
| 19 | 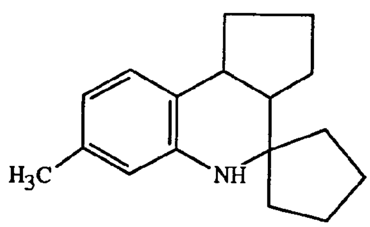 | (116) | Siedepunkt: 175-180° bei einem Druck von $10^{-1}$ mbar |
| 20 | 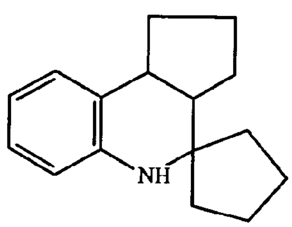 | (117) | Siedepunkt: 165-170° bei einem Druck von 0,7 mbar |

| Bsp. | Octahydrophenanthridinverbindungen und verwandte Verbindungen | | Siede- bzw. Schmelzpunkt |
|---|---|---|---|
| 21 | 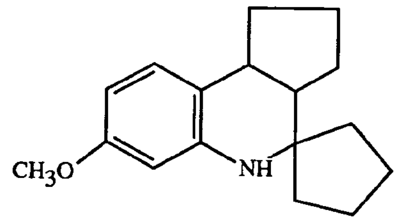 | (118) | Siedepunkt: 170-175° bei einem Druck von 0,4 mbar |

<u>Beispiel 22:</u> Bei Raumtemperatur werden 47,8 Teile der gemäss Beispiel 10 hergestellten Verbindung der Formel (107) in 180 Teile absolutem Toluol gelöst und in einem Sulfierkolben vorgelegt. Nach Zugabe von 42,4 Teilen Diethylsulfat und 6,04 Teilen feingepulvertem Magnesiumoxid wird 10 Stunden bei einer Temperatur von 105 bis 110° gerührt. Nach dem Abkühlen werden die Magnesiumsalze abgesaugt und mit 50 Teilen Toluol ausgewaschen. Die vereinigten Toluolphasen werden anschliessend mit 31,3 Teilen Salzsäurelösung (32%) versetzt und 2 Stunden bei einer Temperatur von 70° gerührt. Nach dem Abkühlen wird das ausgefallene Produkt abgesaugt und mit Toluol nachgewaschen. Nach Trocknung an der Luft erhält man 39,6 Teile weisser Kristalle, die der Verbindung der Formel

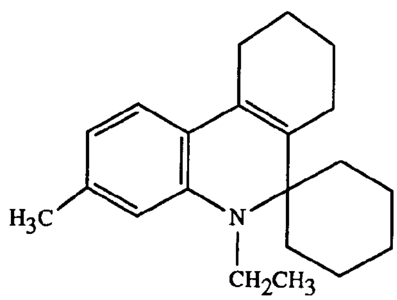

(119)

entsprechen und als Hydrochlorid vorliegen. Die als Hydrochlorid vorliegende Verbindung der Formel (119) weist eine flüssigchromatographisch bestimmte Reinheit von 97% und einen Schmelzpunkt von 189 bis 190° auf.

<u>Beispiele 23 bis 30:</u> Verfährt man wie in Beispiel 22 angegeben, verwendet jedoch statt 47,8 Teile der gemäss Beispiel 10 hergestellten Verbindung der Formel (107) eine äquimolare Menge einer der gemäss den Beispielen 4 bis 9 oder 11 oder 12 erhaltenen Verbindungen, so erhält man die in Tabelle 3 in Spalte 2 angegebenen Hexahydrophenanthridinverbindungen bzw. die angegebenen mit Hexahydrophenanthridin verwandten Verbindungen. Für einige dieser Verbindungen sind in Spalte 3 die Siede- bzw. Schmelzpunkte angegeben.

Tabelle 3

| Bsp. | Hexahydrophenanthridinverbindungen und verwandte Verbindungen | | Siede- bzw. Schmelzpunkt |
|---|---|---|---|
| 23 | 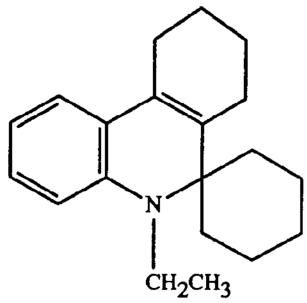 | (120) | |
| 24 | 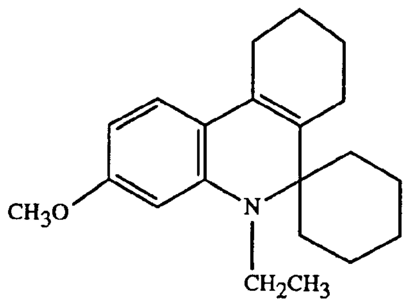 | (121) | |
| 25 | 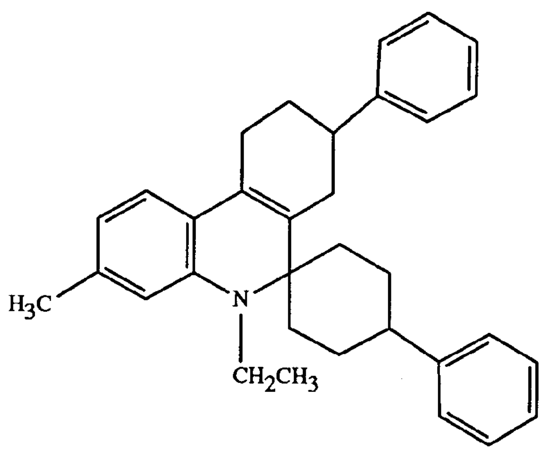 | (122) | Schmelzpunkt: 190° |

| Bsp. | Hexahydrophenanthridinverbindungen und verwandte Verbindungen | | Siede- bzw. Schmelzpunkt |
|---|---|---|---|
| 26 | 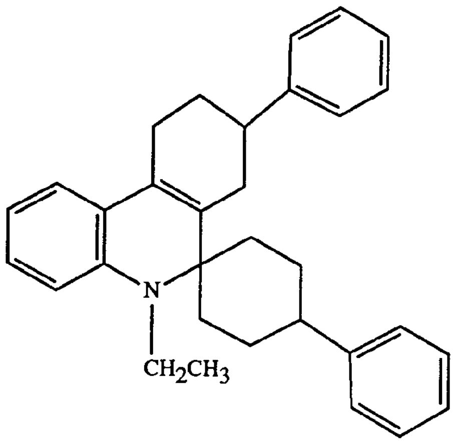 | (123) | |
| 27 | 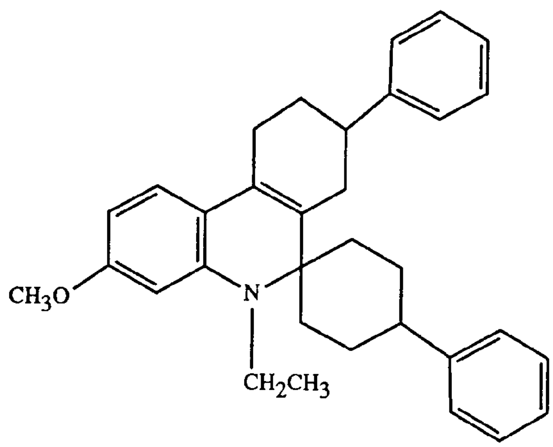 | (124) | |
| 28 | 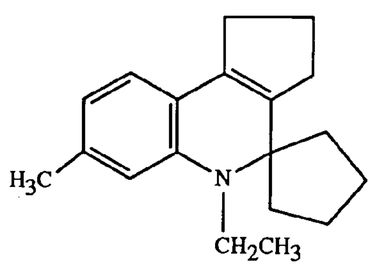 | (125) | Siedepunkt: 175-180° bei einem Druck von 0,1 mbar |

| Bsp. | Hexahydrophenanthridinverbindungen und verwandte Verbindungen | | Siede- bzw. Schmelzpunkt |
|---|---|---|---|
| 29 | 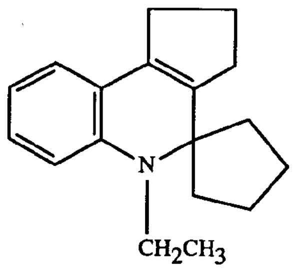 | (126) | |
| 30 | 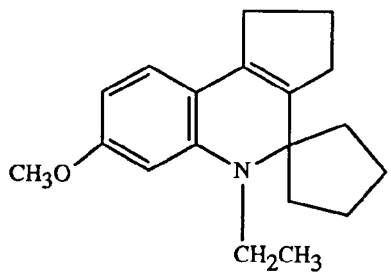 | (127) | |

Beispiel 31: 72 Teile der gemäss Beispiel 28 hergestellten Verbindung der Formel (125) werden in 720 Teilen absolutem Aethanol gelöst und nach Zugabe von 29 Teilen Raney-Nickel 39 Stunden bei einer Temperatur von 50 bis 60° und einem konstanten Druck von 5 bar hydriert. Nach der Aufnahme der theoretischen Wasserstoffmenge wird der Katalysator abfiltriert und das Aethanol im Wasserstrahlvakuum abgezogen. Nach Destillation im Hachvakuum bei einer Temperatur von 145 bis 165° und einem Druck von $4 \cdot 10^{-2}$ mbar erhält man 66,5 Teile einer leicht grünstichigen Flüssigkeit, die der Verbindung der Formel

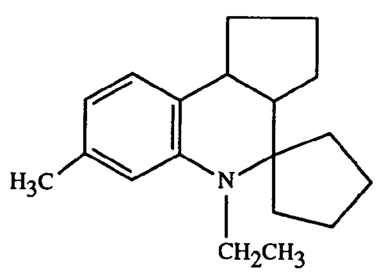

(128)

entspricht. Die Verbindung der Formel (128) weist eine gaschromatographisch bestimmte Reinheit von 92% auf.

Beispiele 32 und 33: Verfährt man wie in Beispiel 31 angegeben verwendet jedoch statt 72 Teile der gemäss Beispiel 28 hergestellten Verbindung der Formel (125) eine äquimolare Menge einer der gemäss Beispiel 29 oder 30 erhaltenen Verbindungen, so erhält man die Verbindungen der Formeln

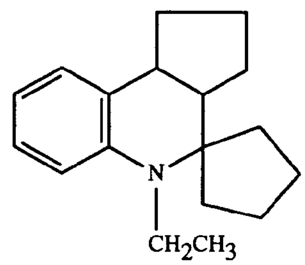

(129) und

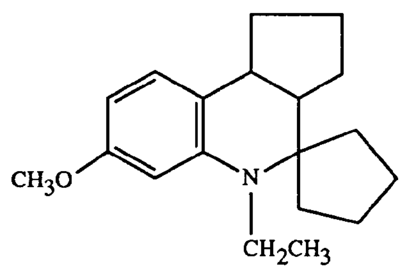

(130).

Beispiel 34: Eine Suspension von 4,7 Teilen 2-Amino-4′-methyldiphenylsulfon-4-(2″-sulfonaphthalid-1″,5″-disulfonsäure), 0,37 Teilen Natriumnitrit und 15 Teilen Wasser wird durch Zugabe von 2,5 Teilen 4-normaler Natriumhydroxid-Lösung bei einer Temperatur von 50° in eine Lösung überführt. Die auf diese Weise hergestellte Lösung wird unter Rühren in ein Gemisch von 22,5 Teilen Eis und 7,5 Teilen konzentrierter Salzsäure (32%) so eingetropft, dass die maximale Innentemperatur bei 5° liegt. Nach Zugabe von 30 Teilen kaltem Wasser wird noch 2 Stunden bei einer Temperatur von 3 bis 5° nachgerührt und der Nitritüberschuss durch Zugabe von 0,35 Teilen Sulfaminsäure zerstört. Danach werden 1,4 Teile der gemäss Beispiel 31 erhaltenen Verbindung der Formel (128) bei ca. 5° innerhalb einer Minute zugetropft und es wird bei einer Temperatur von 3 bis 5° 30 Minuten nachgerührt. Anschliessend gibt man 6 Teile Natriumacetat x $3H_2O$ zu und rührt 3 Stunden bei einer Temperatur von 3 bis 5°. Nach langsamer Zugabe einer Lösung von 6 Teilen Natriumacetat x $3H_2O$ und 1,5 Teilen Natriumcarbonat in 15 Teilen Wasser innerhalb eines Zeitraumes von 20 Minuten wird das Reaktionsgemisch auf 100 Teile einer gesättigten Natriumchlorid-Lösung ausgetragen. Nach Filtrarion und Trocknung im Vakuum bei einer Temperatur von 50° erhält man 5 Teile eines bräunlichen Pulers, das in Form der freien Säure der Verbindung der Formel

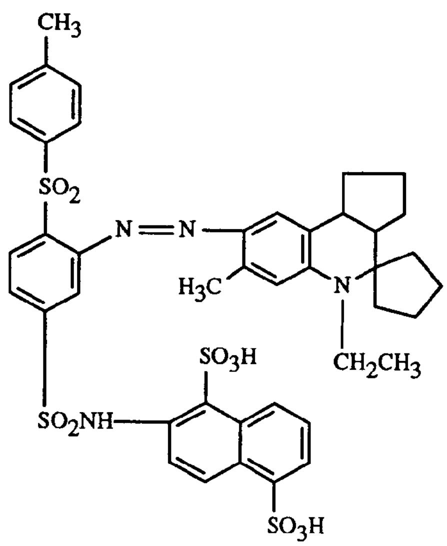

(131)

entspricht. Die Verbindung der Formel (131) färbt natürliches und synthetisches Polyamidmaterial in blaustichig roten Farbtönen.

Beispiele 35 bis 68: Verfährt man wie in Beispiel 34 angegeben, verwendet jedoch gegebenenfalls statt 4,7 Teile 2-Amino-4′-methyldiphenylsulfon-4-(2″-sulfonaphthalid-1″,5″-disulfonsäure) eine äquimolare Menge

einer der in Tabelle 4 in Spalte 2 in Form der freien Säuren angegebenen Diazokomponenten und statt 1,4 Teile der gemäss Beispiel 31 erhaltenen Verbindung der Formel (128) eine äquimolare Menge einer der in Tabelle 4 in Spalte 3 angegebenen Kupplungskomponenten und gibt gegebenenfalls statt der ungelösten Kupplungskomponente eine Lösung der Kupplungskomponente in Tetrahydrofuran zu, so erhält man analoge Farbstoffe, die natürliches und synthetisches Polyamidmaterial in den in Spalte 4 angegebenen Farbtönen färben.

Tabelle 4

| Bsp. | Diazokomponente | Kupplungskomponente | Farbton auf natürlichem und synthetischem Polyamid |
|---|---|---|---|
| 35 | 2-Amino-4'-methyldiphenylsulfon-4-(2''-sulfonaphthalid-1'',5''-disulfonsäure) | 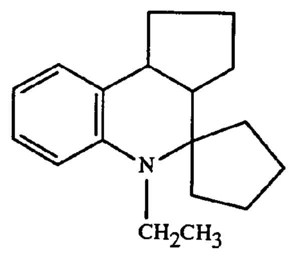 | blaustichig rot |
| 36 | 2-Amino-4'-methyldiphenylsulfon-4-(2''-sulfonaphthalid-1'',5''-disulfonsäure) | 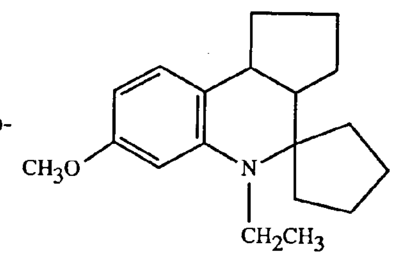 | blaustichig rot |
| 37 | 2-Amino-4'-methyldiphenylsulfon-4-(2''-sulfonaphthalid-1'',5''-disulfonsäure) | 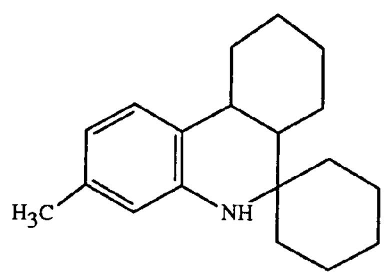 | rot |
| 38 | 2-Amino-4'-methyldiphenylsulfon-4-(2''-sulfonaphthalid-1'',5''-disulfonsäure) | 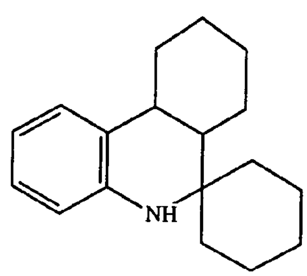 | rot |

| Bsp. | Diazokomponente | Kupplungs-komponente | Farbton auf natürlichem und synthetischem Polyamid |
|---|---|---|---|
| 39 | 2-Amino-4'-methyldiphenylsulfon-4-(2''-sulfonaphthalid-1'',5''-disulfonsäure) | 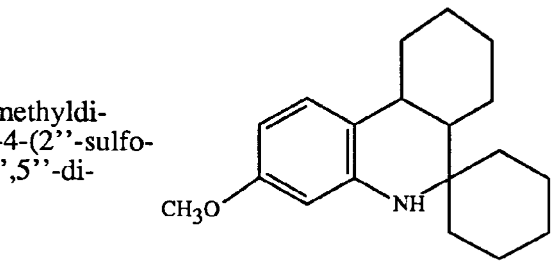 | rot |
| 40 | 2-Amino-4'-methyldiphenylsulfon-4-(2''-sulfonaphthalid-1'',5''-disulfonsäure) | 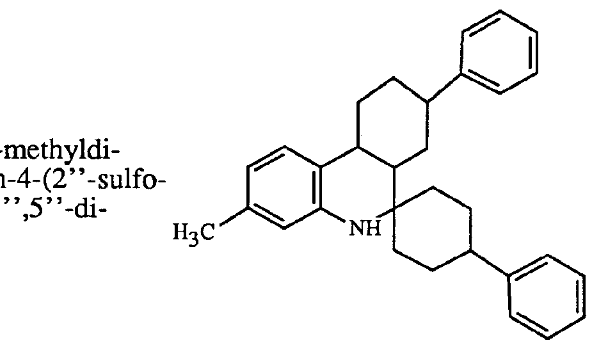 | rot |
| 41 | 2-Amino-4'-methyldiphenylsulfon-4-(2''-sulfonaphthalid-1'',5''-disulfonsäure) | 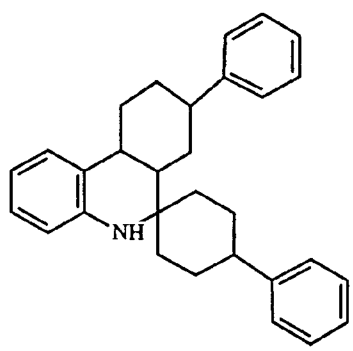 | rot |
| 42 | 2-Amino-4'-methyldiphenylsulfon-4-(2''-sulfonaphthalid-1'',5''-disulfonsäure) | 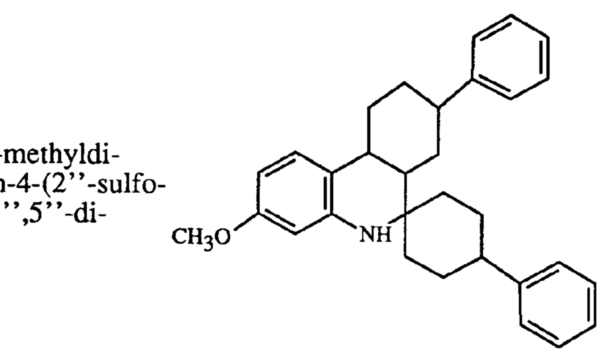 | rot |

| Bsp. | Diazokomponente | Kupplungs-komponente | Farbton auf natürlichem und synthetischem Polyamid |
|---|---|---|---|
| 43 | 2-Amino-4'-methyldi-phenylsulfon-4-(2''-sulfo-naphthalid-5'',7''-di-sulfonsäure) | 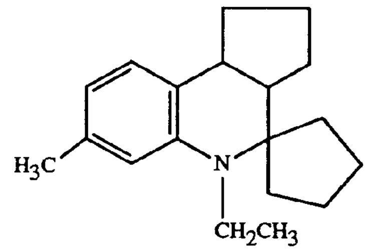 | stark blaustichig rot |
| 44 | 2-Amino-4'-methyldi-phenylsulfon-4-(2''-sulfo-naphthalid-5'',7''-di-sulfonsäure) | 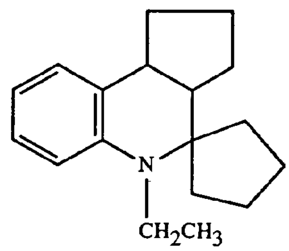 | blaustichig rot |
| 45 | 2-Amino-4'-methyldi-phenylsulfon-4-(2''-sulfo-naphthalid-5'',7''-di-sulfonsäure) | 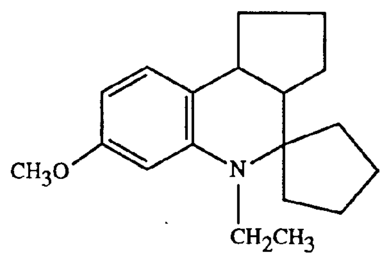 | blaustichig rot |
| 46 | 2-Amino-4'-methyldi-phenylsulfon-4-(2''-sulfo-naphthalid-5'',7''-di-sulfonsäure) | 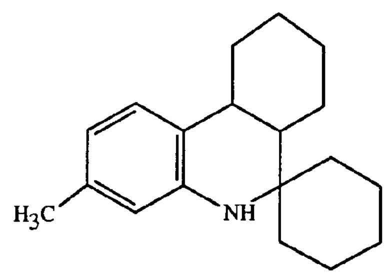 | rot |
| 47 | 2-Amino-4'-methyldi-phenylsulfon-4-(2''-sulfo-naphthalid-5'',7''-di-sulfonsäure) | 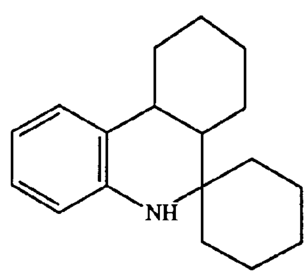 | rot |

| Bsp. | Diazokomponente | Kupplungskomponente | Farbton auf natürlichem und synthetischem Polyamid |
|---|---|---|---|
| 48 | 2-Amino-4'-methyldiphenylsulfon-4-(2''-sulfonaphthalid-5'',7''-disulfonsäure) | 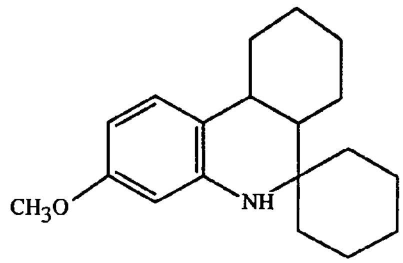 | rot |
| 49 | 2-Amino-4'-methyldiphenylsulfon-4-(2''-sulfonaphthalid-5'',7''-disulfonsäure) | 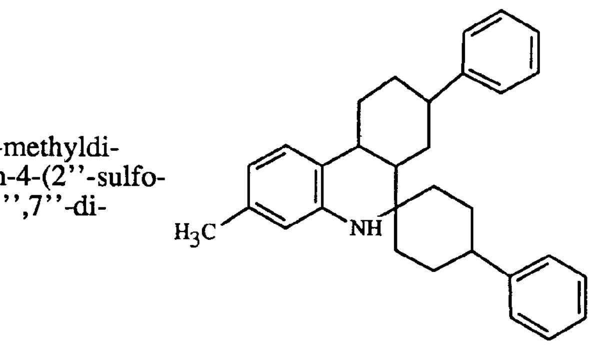 | rot |
| 50 | 2-Amino-4'-methyldiphenylsulfon-4-(2''-sulfonaphthalid-5'',7''-disulfonsäure) | 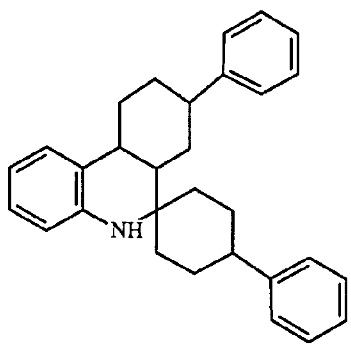 | rot |
| 51 | 2-Amino-4'-methyldiphenylsulfon-4-(2''-sulfonaphthalid-5'',7''-disulfonsäure) | 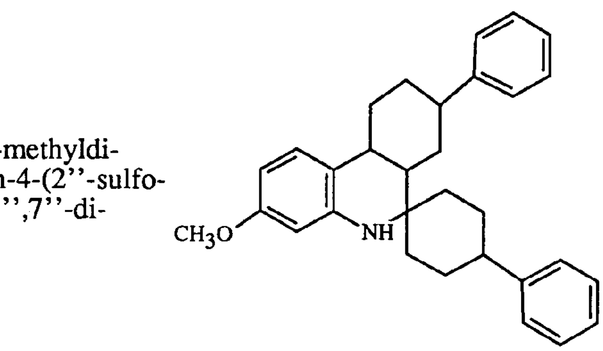 | rot |

| Bsp. | Diazokomponente | Kupplungskomponente | Farbton auf natürlichem und synthetischem Polyamid |
|---|---|---|---|
| 52 | 2-Amino-4'-chlorodiphenylsulfon-4-(2''-sulfonaphthalid-1'',5''-disulfonsäure) | 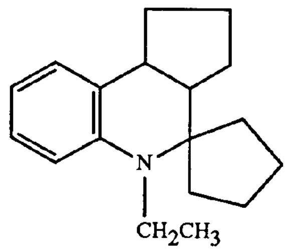 | blaustichig rot |
| 53 | 2-Amino-4'-chlorodiphenylsulfon-4-(2''-sulfonaphthalid-1'',5''-disulfonsäure) | 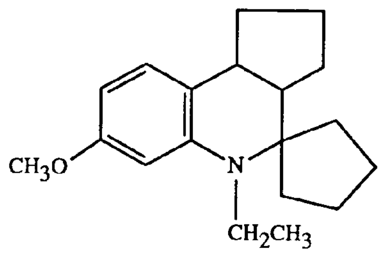 | blaustichig rot |
| 54 | 2-Amino-4'-chlorodiphenylsulfon-4-(2''-sulfonaphthalid-1'',5''-disulfonsäure) | 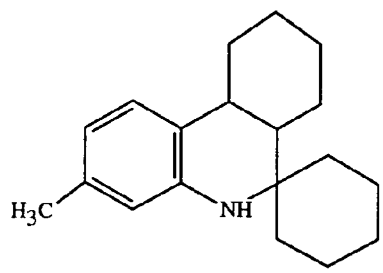 | rot |
| 55 | 2-Amino-4'-chlorodiphenylsulfon-4-(2''-sulfonaphthalid-1'',5''-disulfonsäure) | 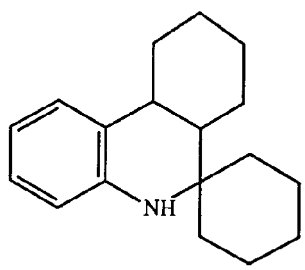 | rot |

| Bsp. | Diazokomponente | Kupplungs-komponente | Farbton auf natürlichem und synthetischem Polyamid |
|---|---|---|---|
| 56 | 2-Amino-4'-chlorodiphenylsulfon-4-(2''-sulfonaphthalid-1'',5''-disulfonsäure) | 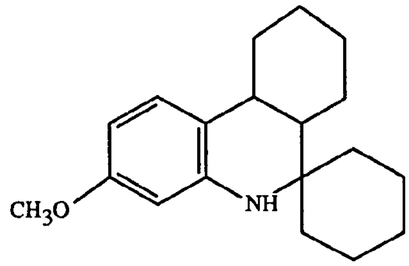 | rot |
| 57 | 2-Amino-4'-chlorodiphenylsulfon-4-(2''-sulfonaphthalid-1'',5''-disulfonsäure) | 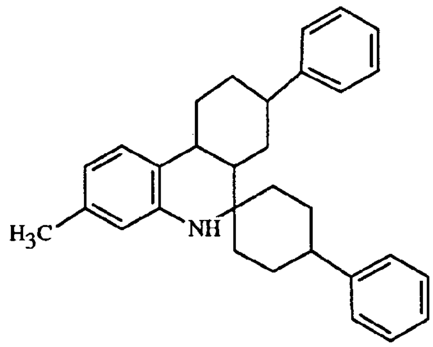 | rot |
| 58 | 2-Amino-4'-chlorodiphenylsulfon-4-(2''-sulfonaphthalid-1'',5''-disulfonsäure) | 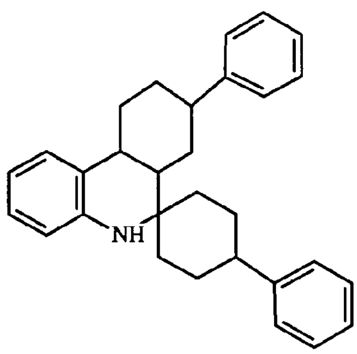 | rot |
| 59 | 2-Amino-4'-chlorodiphenylsulfon-4-(2''-sulfonaphthalid-1'',5''-disulfonsäure) | 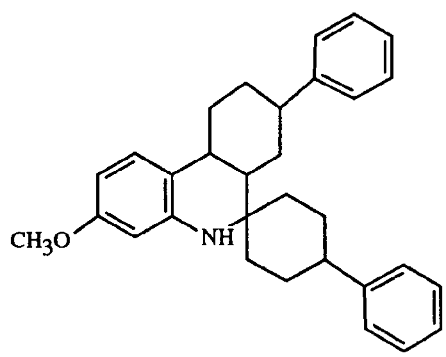 | rot |

| Bsp. | Diazokomponente | Kupplungs-komponente | Farbton auf natürlichem und synthetischem Polyamid |
|---|---|---|---|
| 60 | 2-Amino-4'-chlorodiphenylsulfon-4-(2''-sulfonaphthalid-5'',7''-disulfonsäure) | 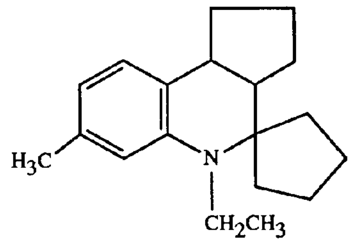 | stark blaustichig rot |
| 61 | 2-Amino-4'-chlorodiphenylsulfon-4-(2''-sulfonaphthalid-5'',7''-disulfonsäure) | 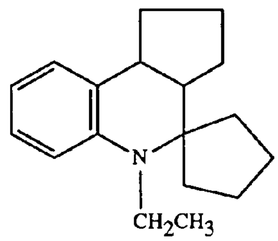 | blaustichig rot |
| 62 | 2-Amino-4'-chlorodiphenylsulfon-4-(2''-sulfonaphthalid-5'',7''-disulfonsäure) | 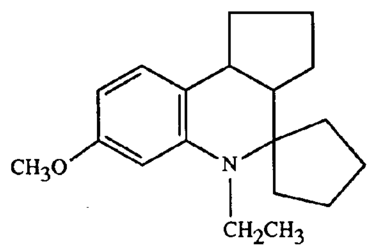 | blaustichig rot |
| 63 | 2-Amino-4'-chlorodiphenylsulfon-4-(2''-sulfonaphthalid-5'',7''-disulfonsäure) | 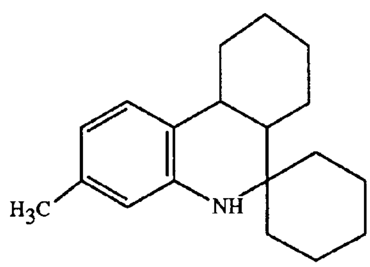 | rot |
| 64 | 2-Amino-4'-chlorodiphenylsulfon-4-(2''-sulfonaphthalid-5'',7''-disulfonsäure) | 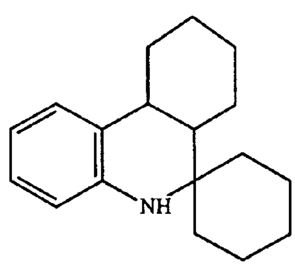 | rot |

| Bsp. | Diazokomponente | Kupplungskomponente | Farbton auf natürlichem und synthetischem Polyamid |
|---|---|---|---|
| 65 | 2-Amino-4'-chlorodiphenylsulfon-4-(2"-sulfonaphthalid-5",7"-disulfonsäure) | 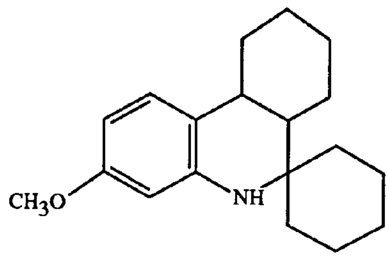 | rot |
| 66 | 2-Amino-4'-chlorodiphenylsulfon-4-(2"-sulfonaphthalid-5",7"-disulfonsäure) | 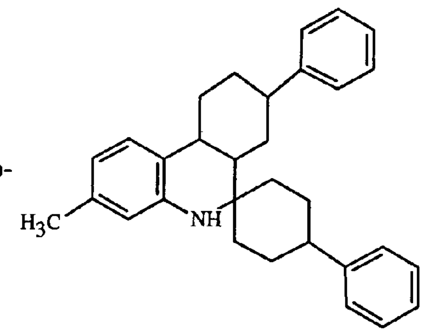 | rot |
| 67 | 2-Amino-4'-chlorodiphenylsulfon-4-(2"-sulfonaphthalid-5",7"-disulfonsäure) | 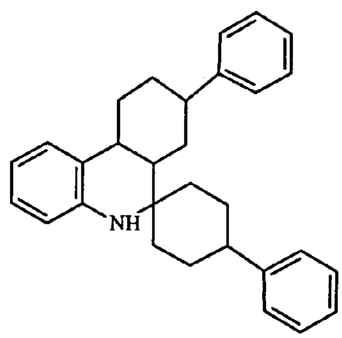 | rot |
| 68 | 2-Amino-4'-chlorodiphenylsulfon-4-(2"-sulfonaphthalid-5",7"-disulfonsäure) | 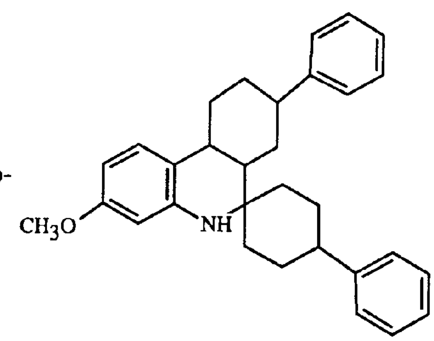 | rot |

Färbevorschrift I

Man färbt 10 Teile Polyamid-6,6-Gewebe in 500 Teilen einer wässrigen Flotte, die 2 g/l Ammonacetat enthält und mit Essigsäure auf pH 5 gestellt wird. Der Anteil des Farbstoffs gemäss Beispiel 34 beträgt 0,7 % bezogen auf das Fasergewicht. Die Färbedauer bei einer Temperatur von 98° beträgt 30 bis 90 Minuten. Das

gefärbte Polyamid-6,6-Gewebe wird anschliessend herausgenommen und wie üblich gewaschen und getrocknet.

Man erhält ein blaustichig rot gefärbtes Polyamid-6,6-Gewebe, das eine reine Nuance und gute Gesamtechtheiten aufweist.

Färbevorschrift II

Man färbt 10 Teile Polyamid-6,6-Gewebe in 500 Teilen einer wässrigen Flotte, die 1 g/l Mononatriumphosphat enthält und mit Dinatriumphosphat auf pH 6 gestellt wird. Der Anteil des Farbstoffes gemäss Beispiel 37 beträgt 1 %, bezogen auf das Fasergewicht. Die Färbedauer bei einer Temperatur von 98° beträgt 30 bis 90 Minuten. Das gefärbte Polyamid-6,6-Gewebe wird anschliessend herausgenommen und wie üblich gewaschen und getrocknet.

Man erhält ein rot gefärbtes Polyamid-6,6-Gewebe, das eine reine Nuance und gute Gesamtechtheiten aufweist.

Färbevorschrift III

Man färbt 10 Teile Wollstück in 500 Teilen einer wässrigen Flotte. Bezogen auf das Fasergewicht betragen die Anteile an Farbstoff 0,45 % gemäss Beispiel 46, an Glaubersalz kalz. 5 % und 80%-iger Essigsäure 2 %. Die Färbedauer bei einer Temperatur von 98° beträgt 30-60 Minuten. Das rot gefärbte, wie üblich gewaschene und getrocknete Wollstück weist sehr gute Allgemeinechtheiten auf.

**Patentansprüche**

1. Azofarbstoffe der Formel

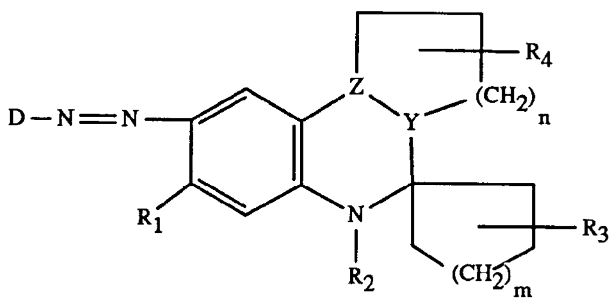

(1),

worin D eine Diazokomponente der Benzol- oder Naphthalinreihe oder der heterocyclischen Reihe ist, $R_1$ Wasserstoff, Halogen oder gegebenenfalls substituiertes $C_1$-$C_8$-Alkyl, $C_1$-$C_8$-Alkoxy, Phenoxy oder $C_2$-$C_8$-Alkanoylamino, $R_2$ Wasserstoff oder gegebenenfalls substituiertes $C_1$-$C_8$-Alkyl ist, $R_3$ und $R_4$ unabhängig voneinander Wasserstoff, Halogen oder gegebenenfalls substituiertes $C_1$-$C_8$-Alkyl oder Phenyl sind,

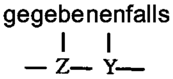

den Rest der Formel

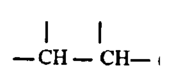

oder

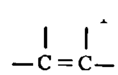

bedeutet und m und n unabhängig voneinander die Zahl 1 oder 2 sind.

2. Azofarbstoffe gemäss Anspruch 1, worin D gegebenenfalls substituiertes Thienyl, Thiazolyl, Isothiazolyl, 1,2,4-Thiadiazolyl, 1,3,4-Thiadiazolyl, Benzthiazolyl, Benzisothiazolyl, Benzoxazolyl, Pyrazolyl, Imidazolyl, 1,2,3-Triazolyl, 1,2,4-Triazolyl, Thiophenyl, Benzthiophenyl, Tetrahydrobenzthiophenyl, 7-Oxotetrahydrobenzo[b]thiophenyl, Pyridinyl, Indazolyl, Phenyl oder Naphthyl ist.

3. Azofarbstoffe gemäss einem der Ansprüche 1 und 2, worin $R_1$ Wasserstoff, $C_1$-$C_4$-Alkyl, insbesondere Methyl, oder $C_1$-$C_4$-Alkoxy, insbesondere Methoxy, ist.

4. Azofarbstoffe gemäss einem der Ansprüche 1 bis 3, worin $R_2$ Wasserstoff oder gegebenenfalls durch Phenyl substituiertes $C_1$-$C_4$-Alkyl, insbesondere Aethyl, ist.

5. Azofarbstoffe gemäss einem der Ansprüche 1 bis 4, worin $R_3$ und $R_4$ unabhängig voneinander Wasserstoff oder gegebenenfalls substituiertes Phenyl sind.

6. Azofarbstoffe gemäss einem der Ansprüche 1 bis 5, worin n und m beide die Zahl 1 oder n und m beide die Zahl 2 bedeuten.

7. Azofarbstoffe gemäss einem der Ansprüche 1 bis 6, worin

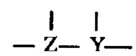

den Rest der Formel

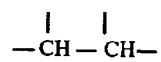

bedeutet.

8. Azofarbstoffe gemäss einem der Ansprüche 1 bis 7, worin $R_1$ Wasserstoff, Methyl oder Methoxy, $R_2$ Wasserstoff oder Äthyl und $R_3$ und $R_4$ unabhängig voneinander Wasserstoff oder Phenyl sind und

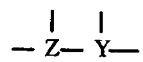

den Rest der Formel

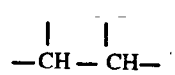

bedeutet.

9. Verfahren zur Herstellung von Azofarbstoffen gemäss Anspruch 1, dadurch gekennzeichnet, dass man ein Amin der Formel

D-$NH_2$ (2)

diazotiert und auf eine Kupplungskomponente der Formel

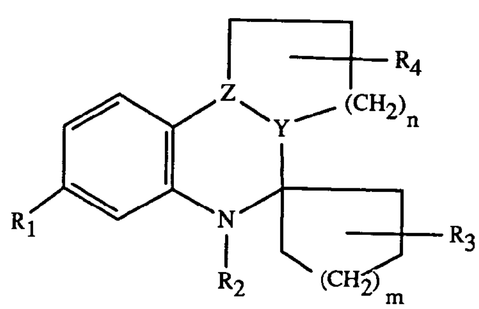

(3)

kuppelt, wobei D, $R_1$, $R_2$, $R_3$, $R_4$

$$,-\overset{|}{Z}-\overset{|}{Y}-$$

n und m die in Anspruch 1 angegebenen Bedeutungen haben.

**10.** Verbindungen der Formel

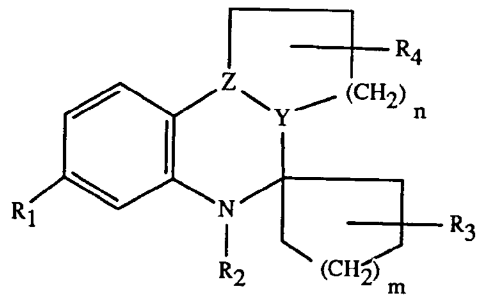

(4),

worin

$$-\overset{|}{Z}-\overset{|}{Y}-$$

den Rest der Formel

$$-\overset{|}{CH}-\overset{|}{CH}-$$

bedeutet, $R_1$ Wasserstoff, Halogen oder gegebenenfalls substituiertes $C_1$-$C_8$-Alkyl, $C_1$-$C_8$-Alkoxy, Phenoxy oder $C_2$-$C_8$-Alkanoylamino, $R_2$ Wasserstoff oder gegebenenfalls substituiertes $C_1$-$C_8$-Alkyl ist, $R_3$ und $R_4$ unabhängig voneinander Wasserstoff, Halogen oder gegebenenfalls substituiertes $C_1$-$C_8$-Alkyl oder Phenyl und m und n unabhängig voneinander die Zahl 1 oder 2 sind, oder worin

$$-\overset{|}{Z}-\overset{|}{Y}-$$

den Rest der Formel

$$-\overset{|}{C}=\overset{|}{C}-$$

bedeutet, $R_1$ Halogen oder gegebenenfalls substituiertes $C_1$-$C_8$-Alkyl, $C_1$-$C_8$-Alkoxy, Phenoxy oder $C_2$-$C_8$-Alkanoylamino ist und $R_2$, $R_3$, $R_4$, m und n die unter Formel (4) angegebenen Bedeutungen haben.

**11.** Verfahren zur Herstellung von Verbindungen gemäss Anspruch 10, dadurch gekennzeichnet, dass man eine Verbindung der Formel

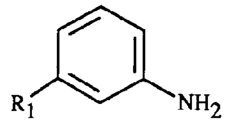

(5),

mit den Verbindungen der Formeln

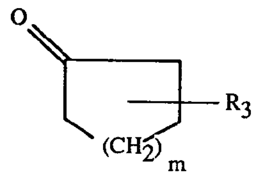 (6) und 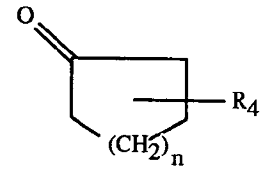 (7)

oder mit einer Verbindung der Formel

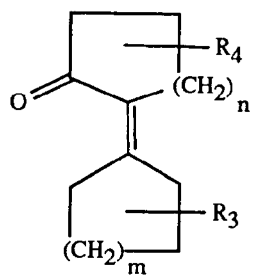

(8)

zu einer Verbindung der Formel

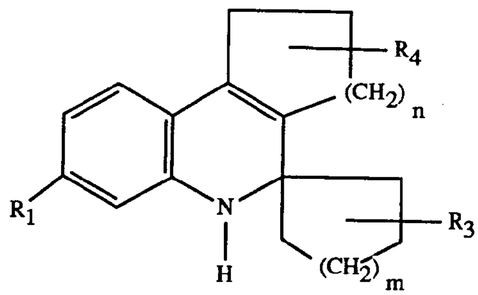

(9)

umsetzt, gegebenenfalls die Verbindung der Formel (9) zu einer Verbindung der Formel

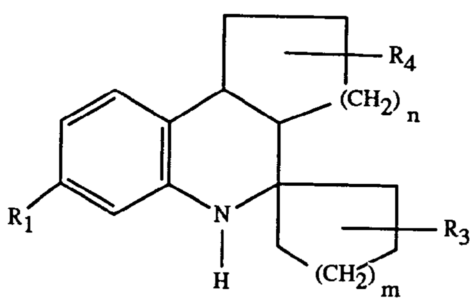

(10)

hydriert und gegebenenfalls die Verbindung der Formel (9) oder (10) mit einer Verbindung der Formel

$$(R_2)_2X \quad (11)$$

oder

$$R_2X \quad (12)$$

worin X eine ein- oder zweiwertige anionische Abgangsgruppe ist, umsetzt, wobei $R_1$, $R_2$, $R_3$, $R_4$, n und m die in Anspruch 1 angegebenen Bedeutungen haben.

**12.** Verwendung der Azofarbstoffe gemäss den Ansprüchen 1 bis 8, bzw. der gemäss Anspruch 9 erhaltenen Azofarbstoffe zum Färben und Bedrucken von vorzugsweise stickstoffhaltigen oder hydroxygruppenhaltigen Fasermaterialien.

**13.** Verwendung der Verbindungen gemäss Anspruch 10 bzw. der gemäss Anspruch 11 erhaltenen Verbindungen als Antioxidations- oder Lichtschutzmittel.

**Patentansprüche Für folgenden Vertragsstaat: ES**

1. Verfahren zur Herstellung von Azofarbstoffen der Formel

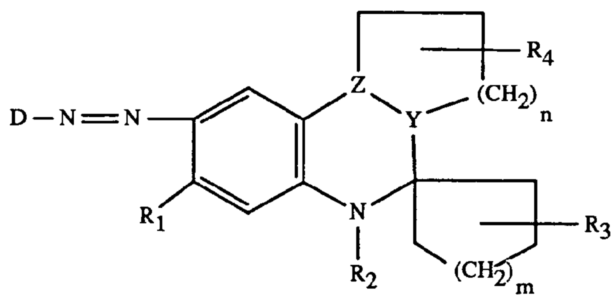

(1),

worin D eine Diazokomponente der Benzol- oder Naphthalinreihe oder der heterocyclischen Reihe ist, $R_1$ Wasserstoff, Halogen oder gegebenenfalls substituiertes $C_1$-$C_8$-Alkyl, $C_1$-$C_8$-Alkoxy, Phenoxy oder $C_2$-$C_8$-Alkanoylamino, $R_2$ Wasserstoff oder gegebenenfalls substituiertes $C_1$-$C_8$-Alkyl ist, $R_3$ und $R_4$ unabhängig voneinander Wasserstoff, Halogen oder gegebenenfalls substituiertes $C_1$-$C_8$-Alkyl oder Phenyl sind

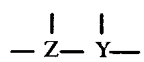

den Rest der Formel

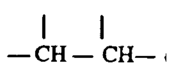

oder

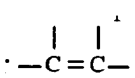

bedeutet und m und n unabhängig voneinander die Zahl 1 oder 2 sind, dadurch gekennzeichnet, dass man ein Amin der Formel

$D\text{-}NH_2$ (2)

diazotiert und auf eine Kupplungskomponente der Formel

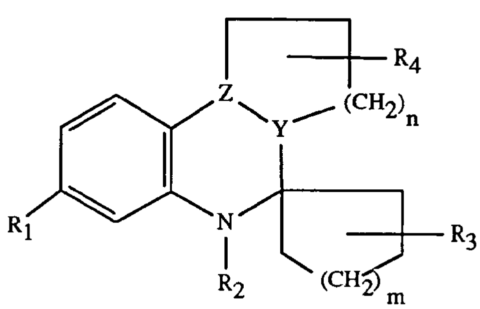

(3)

kuppelt, wobei D, $R_1$, $R_2$, $R_3$, $R_4$,

$$,-\overset{|}{Z}-\overset{|}{Y}-\ ,$$

n und m die unter Formel (1) angegebenen Bedeutungen haben.

**2.** Verfahren gemäss Anspruch 1, dadurch gekennzeichnet, dass man eine Diazokomponente der Formel (2) verwendet, worin D gegebenenfalls substituiertes Thienyl, Thiazolyl, Isothiazolyl, 1,2,4-Thiadiazolyl, 1,3,4-Thiadiazolyl, Benzthiazolyl, Benzisothiazolyl, Benzoxazolyl, Pyrazolyl, Imidazolyl, 1,2,3-Triazolyl, 1,2,4-Triazolyl, Thiophenyl, Benzthiophenyl, Tetrahydrobenzthiophenyl, 7-Oxotetrahydrobenzo[b]thiophenyl, Pyridinyl, Indazolyl, Phenyl oder Naphthyl ist.

**3.** Verfahren gemäss einem der Ansprüche 1 und 2, dadurch gekennzeichnet, dass man eine Kupplungskomponente der Formel (3) verwendet, worin $R_1$ Wasserstoff, $C_1$-$C_4$-Alkyl, insbesondere Methyl, oder $C_1$-$C_4$-Alkoxy, insbesondere Methoxy, ist.

**4.** Verfahren gemäss einem der Ansprüche 1 bis 3, dadurch gekennzeichnet, dass man eine Kupplungskomponente der Formel (3) verwendet, worin $R_2$ Wasserstoff oder gegebenenfalls durch Phenyl substituiertes $C_1$-$C_4$-Alkyl, insbesondere Aethyl, ist.

**5.** Verfahren gemäss einem der Ansprüche 1 bis 4, dadurch gekennzeichnet, dass man eine Kupplungskomponente der Formel (3) verwendet, worin $R_3$ und $R_4$ unabhängig voneinander Wasserstoff oder gegebenenfalls substituiertes Phenyl sind.

**6.** Verfahren gemäss einem der Ansprüche 1 bis 5, dadurch gekennzeichnet, dass man eine Kupplungskomponente der Formel (3) verwendet, worin n und m beide die Zahl 1 oder n und m beide die Zahl 2 bedeuten.

**7.** Verfahren gemäss einem der Ansprüche 1 bis 6, dadurch gekennzeichnet, dass man eine Kupplungskomponente der Formel (3) verwendet, worin

$$-\overset{|}{Z}-\overset{|}{Y}-$$

den Rest der Formel

$$-\overset{|}{CH}-\overset{|}{CH}-$$

bedeutet.

**8.** Verfahren gemäss einem der Ansprüche 1 bis 7, dadurch gekennzeichnet, dass man eine Kupplungskomponente der Formel (3) verwendet, worin $R_1$ Wasserstoff, Methyl oder Methoxy, $R_2$ Wasserstoff oder Äthyl und $R_3$ und $R_4$ unabhängig voneinander Wasserstoff oder Phenyl sind und

$$-\overset{|}{Z}-\overset{|}{Y}-$$

den Rest der Formel

$$-\overset{|}{CH}-\overset{|}{CH}-$$

bedeutet.

**9.** Verfahren zur Herstellung von Verbindungen der Formel

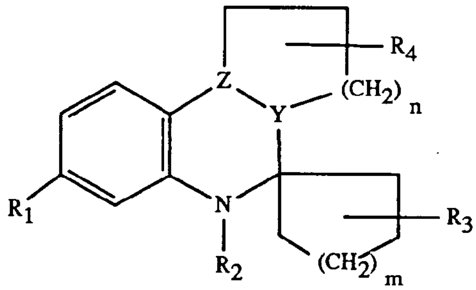

(4),

worin

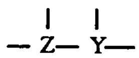

den Rest der Formel

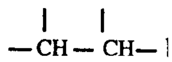

bedeutet, $R_1$ Wasserstoff, Halogen oder gegebenenfalls substituiertes $C_1$-$C_8$-Alkyl, $C_1$-$C_8$-Alkoxy, Phenoxy oder $C_2$-$C_8$-Alkanoylamino, $R_2$ Wasserstoff oder gegebenenfalls substituiertes $C_1$-$C_8$-Alkyl ist, $R_3$ und $R_4$ unabhängig voneinander Wasserstoff, Halogen oder gegebenenfalls substituiertes $C_1$-$C_8$-Alkyl oder Phenyl und m und n unabhängig voneinander die Zahl 1 oder 2 sind oder worin

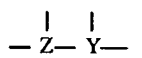

den Rest der Formel

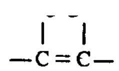

bedeutet, $R_1$ Halogen oder gegebenenfalls substituiertes $C_1$-$C_8$-Alkyl, $C_1$-$C_8$-Alkoxy, Phenoxy oder $C_2$-$C_8$-Alkanoylamino ist und $R_2$, $R_3$, $R_4$, m und n die unter Formel (4) angegebenen Bedeutungen haben, dadurch gekennzeichnet, dass man eine Verbindung der Formel

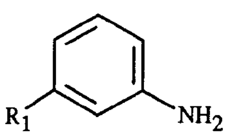

(5),

mit den Verbindungen der Formeln

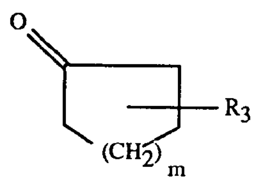

(6) und

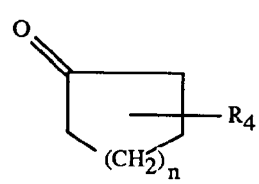

(7)

oder mit einer Verbindung der Formel

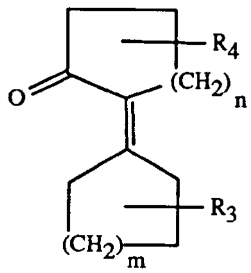

(8)

zu einer Verbindung der Formel

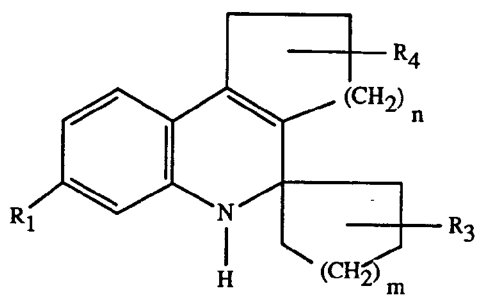

(9)

umsetzt, gegebenenfalls die Verbindung der Formel (9) zu einer Verbindung der Formel

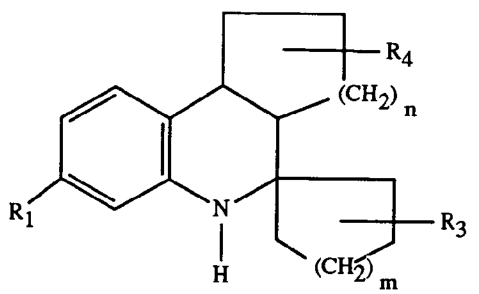

(10)

hydriert und gegebenenfalls die Verbindung der Formel (9) oder (10) mit einer Verbindung der Formel

$(R_2)_2X$ (11)

oder

$R_2X$ (12),

worin X eine ein- oder zweiwertige anionische Abgangsgruppe ist, umsetzt, wobei $R_1$, $R_2$, $R_3$, $R_4$, n und m die unter Formel (4) angegebenen Bedeutungen haben.

**10.** Verwendung der gemäss den Ansprüchen 1 bis 8 erhaltenen Azofarbstoffe zum Färben und Bedrucken von vorzugsweise stickstoffhaltigen oder hydroxygruppenhaltigen Fasermaterialien.

**11.** Verwendung der gemäss Anspruch 9 erhaltenen Verbindungen als Antioxidations- oder Lichtschutzmittel.